# EUROPÄISCHE PATENTSCHRIFT

(11) **EP 1 685 098 B1**
(45) Veröffentlichungstag und Bekanntmachung des Hinweises auf die Patenterteilung: **14.04.2010**
(21) Anmeldenummer: 04818381.8
(22) Anmeldetag: 03.11.2004
(51) Int. Cl.: C07C 381/00

(54) **ORTHO-SUBSTITUIERTE PENTAFLUORSULFANYL-BENZOLE, VERFAHREN ZU IHRER HERSTELLUNG SOWIE IHRE VERWENDUNG AIS WERTVOLLE SYNTHESE-ZWISCHENSTUFEN**
ORTHO-SUBSTITUTED PENTAFLUORIDE SULFANYL-BENZENES, METHOD FOR THE PRODUCTION THEREOF AND THE USE THEREOF IN THE FORM OF VALUABLE SYNTHESIS INTERMEDIATE STAGES
PENTAFLUOROSULFANYLBENZENES ORTHO-SUBSTITUES, LEUR PROCEDE DE PRODUCTION ET LEUR UTILISATION COMME INTERMEDIAIRES DE SYNTHESE PRECIEUX

(30) Priorität: 13.11.2003 DE 10353205
(43) Veröffentlichungstag der Anmeldung: 02.08.2006
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE); WECK, Remo, 65779 Kelkheim (DE)
(86) Internationale Anmeldenummer: PCT/EP2004/012394
(87) Internationale Veröffentlichungsnummer: WO 2005/047240

(56) Entgegenhaltungen:
- WO-A-94/21606
- WO-A-94/22817
- DE-A1- 19 748 109
- A. M. SIPYAGIN ET AL.: "Preparation of the first ortho-subsituted pentafluorosulfanylbenzenes" JOURNAL OF FLUORINE CHEMISTRY, Bd. 112, 2001, Seiten 287-295, XP002323728 in der Anmeldung erwähnt

## Beschreibung

Die Chemie der Pentafluorsulfanyl-Derivate hat in den letzten Jahren an Bedeutung zugenommen, insbesondere da neue Herstellungsverfahren gefunden wurden (Tetrahedron 56 (2000) 3399; Organic Letters 4(17) (2002) 3013). Allerdings sind bisher nur sehr wenige Verbindungen bekannt, die an einem Phenylring in ortho-Position zu der Pentafluorsulfanylgruppe andere Substituenten tragen als Wasserstoff und Fluor. Der einzige bekannte Syntheseweg (Journal of Fluorine Chemistry 112 (2001) 287) verwendet teure Reagentien wie AgF₂ und ist mit geringen Ausbeuten behaftet. Die Autoren begründen dies mit der großen Raumerfüllung der Pentafluorsulfanylgruppe, die ortho-Substitution generell sehr erschwert. Diese Meinung wird auch von anderen Autoren geteilt (J. Am. Chem. Soc. 84 (1962) 3064). Es war daher überraschend, dass die elektrophile Substitution in ortho-Position zur Pentafluorsulfanylgruppe gelingt. Man erhält auf diesem neuen Weg neue ortho-substituierte Pentafluorsulfanyl-Benzole, die wertvolle Intermediate, beispielsweise zur Herstellung von Medikamenten, Diagnostika, Flüssigkristallen, Polymeren, Pestiziden, Herbiziden, Fungiziden, Nematiziden, Parasitiziden, Insektiziden, Akariziden und Arthropodiziden darstellen.

Die Erfindung betrifft Pentafluorsulfanyl-Benzole der Formel I 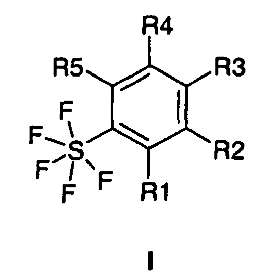 worin bedeuten
- R1: Cl, Br, I, -SO₂R6 oder NO₂;
R6 OH, F, CI, Br, I oder Alkyl mit 1, 2, 3 oder 4 C-Atomen;
- R2 und R4: unabhängig voneinander Wasserstoff, F, Cl, Br, I, -CN, -NR9R10, -OR11, - SR12, COR13, -(SOᵣ)ₛ-(CH₂)ₜ-(CF₂)ᵤ-CF₃, Alkyl mit 1, 2, 3, 4, 5 oder 6 C- Atomen, Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Alkyl mit 1, 2, 3 oder 4 C-Atomen, -(CH₂)ᵥ -(CF₂)_{w}-CF₃, Alkylcarbonyl mit 1, 2, 3 oder 4 C- Atomen, Alkylsulfonyl mit 1, 2, 3 oder 4 C-Atomen;
oder
R9 und R10 bilden zusammen mit dem sie tragenden Stickstoffatom einen Heterocyclus der Formel IIIa: 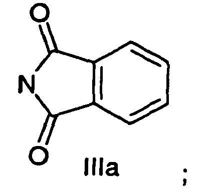
R11 und R12 unabhängig voneinander Wasserstoff, Alkyl mit 1, 2, 3 oder 4 C-Atomen, -(CH₂)ᵥ -(CF₂)_{w}-CF₃, Alkylcarbonyl mit 1, 2, 3 oder 4 C-Atomen, Alkylsulfonyl mit 1, 2, 3 oder 4 C-Atomen;
R13 OH, Alkyl mit 1, 2, 3, 4, 5 oder 6 C-Atomen oder Alkoxy mit 1 2, 3, 4, 5 oder 6 C-Atomen;
s Null;
t und u unabhängig voneinander Null oder 1;
v und w unabhängig voneinander Null oder 1; wobei mindestens einer der Reste R2 und R4 nicht Wasserstoff, Cl, CN, COR13 oder -(SOᵣ)ₛ-(CH₂)ₜ-(CF₂)ᵤ-CF₃ ist;
- R3: Wasserstoff, F, Cl, Br, I, -CN, -NO₂, -COR14, -SO₂CH₃, Alkyl mit 1, 2, 3, 4, 5 oder 6 C-Atomen, Alkoxy mit 1, 2, 3 oder 4 C-Atomen, -Oₓ-(CH₂)_{y}-CF₃,
R14 OH, Alkyl mit 1, 2, 3, 4, 5 oder 6 C-Atomen, Alkoxy mit 1, 2, 3, 4, 5 oder 6 C-Atomen oder -Oₐₐ-(CH₂)_{bb}-CF₃;
x Null oder 1;
y Null, 1, 2 oder 3;
aa Null oder 1;
bb Null, 1, 2 oder 3;
- R5: Wasserstoff oder F;
sowie deren Salze;
wobei Verbindungen der Formel I ausgeschlossen sind, in denen R2 und R4 Cl und R3 F oder Cl bedeuten,
wobei Verbindungen der Formel I ausgeschlossen sind, in denen einer der Substituenten R2 und R4 Cl und der andere der Substituenten R4 und R2 CN und R3 Cl bedeuten und
wobei Verbindungen der Formel I ausgeschlossen sind, in denen R1 NO₂ und die anderen Substituenten Wasserstoff bedeuten.

In einer Ausführungsform sind Verbindungen der Formel I bevorzugt, in denen R1 durch Cl, Br, I, -SO₂R6 , wobei R6 OH, F, Cl, Br, I oder Alkyl mit 1, 2, 3 oder 4 C-Atomen ist, oder -NO₂ beschrieben wird; besonders bevorzugt sind Verbindungen der Formel I, in denen R1 durch Cl, Br, I -SO₂R6 , wobei R6 OH oder Cl ist, oder -NO₂ beschrieben wird; ganz besonders bevorzugt sind Verbindungen der Formel I, in denen R1 durch Cl oder NO₂, insbesondere durch NO₂, beschrieben wird. In einer weiteren Ausführungsform sind Verbindungen der Formel I besonders bevorzugt, in denen R1 durch Cl, Br, I -SO₂R6 beschrieben wird, wobei R6 OH oder Cl ist.

In einer weiteren Ausführungsform sind Verbindungen der Formel I bevorzugt, in denen R2 und R4 unabhängig voneinander durch Wasserstoff, F, Cl, Br, I, -CN, -(SOᵣ)ₛ-(CH₂)ₜ-(CF₂)ᵤ-CF₃, wobei s Null ist und t und u unabhängig voneinander Null oder 1 sind, -NR9R10, -OR11, -SR12, COR13, Alkyl mit 1, 2, 3, 4, 5 oder 6 C-Atomen, Cycloalkyl mit 3, 4, 5, 6, 7 oder 8 C-Atomen, in dem 1, 2, 3 oder 4 Wasserstoff-Atome durch Fluoratome ersetzt sein können, wobei R9 und R10 unabhängig voneinander Alkyl mit 1, 2, 3 oder 4 C-Atomen, -(CH₂)ᵥ -(CF₂)_{w}-CF₃, Alkylcarbonyl mit 1, 2, 3 oder 4 C-Atomen oder Alkylsulfonyl mit 1, 2, 3 oder 4 C-Atomen sind, wobei v und w unabhängig voneinander Null oder 1 sind, oder R9 und R10 bilden zusammen mit dem sie tragenden Stickstoffatom einen Heterocyclus der Formel IIIa: 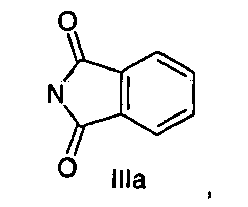 R11 und R12 unabhängig voneinander Wasserstoff, Alkyl mit 1, 2, 3 oder 4 C-Atomen, -(CH₂)ᵥ -(CF₂)_{w}-CF₃, Alkylcarbonyl mit 1, 2, 3 oder 4 C-Atomen oder Alkylsulfonyl mit 1, 2, 3 oder 4 C-Atomen sind, wobei v und w unabhängig voneinander Null oder 1 sind, und wobei R13 OH, Alkyl mit 1, 2, 3, 4, 5 oder 6 C-Atomen oder Alkoxy mit 1, 2, 3, 4, 5 oder 6 C-Atomen bedeutet, beschrieben werden; besonders bevorzugt sind Verbindungen, in denen R2 und R4 unabhängig voneinander durch Wassserstoff, NR9R10 oder COR13 beschrieben werden, wobei R9 und R10 unabhängig voneinander Alkylcarbonyl mit 1, 2, 3 oder 4 C-Atomen, insbesondere Methylcarbonyl, bedeuten oder R9 und R10 bilden zusammen mit dem sie tragenden Stickstoffatom einen Heterocyclus der Formel IIIa,
und wobei R13 Alkoxy mit 1, 2, 3, 4, 5 oder 6 C-Atomen, insbesondere Methoxy, bedeutet.

In einer weiteren Ausführungsform wird einer der Reste R2 und R4 in den Verbindungen der Formel I durch Wasserstoff beschrieben.

In einer weiteren Ausführungsform sind Verbindungen der Formel I bevorzugt, in denen R3 durch Wasserstoff, F, Cl, Br, I, -CN oder -COR14, wobei R14 OH oder Alkoxy mit 1, 2, 3, 4, 5 oder 6 C-Atomen, insbesondere Methoxy, ist, beschrieben wird; besonders bevorzugt sind Verbindungen der Formen I, in denen R3 durch Wasserstoff, CN oder COOCH₃ beschrieben wird.

In einer weiteren Ausführungsform sind Verbindungen der Formel I bevorzugt, in denen R5 durch Wasserstoff oder F beschrieben wird; besonders bevorzugt sind Verbindungen der Formel I, in denen R5 durch Wasserstoff beschrieben wird.

Reste, die mehrfach vorkommen, können gleich oder verschieden sein und unabhängig voneinander die angegebenen Bedeutungen haben.

Enthalten die Substituenten R1 bis R5 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben in allen Verhältnissen vorliegen.

Die vorliegende Erfindung umfasst alle tautomeren Formen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl und Isopropyl. In Alkylresten können ein oder mehrere, zum Beispiel 1, 2, 3, 4 oder 5, Wasserstoffatome durch Fluoratome substituiert sein. Beispiele für solche Fluoralkylreste sind Trifluormethyl, 2,2,2-Trifluorethyl und Pentafluorethyl. Substituierte Alkylreste können in beliebigen Positionen substituiert sein.

Beispiele für Cycloalkylreste sind Cyclopropyl, Cyclobutyl, Cyclopentyl, Cyclohexyl Cycloheptyl oder Cyclooctyl. In Cycloalkylresten können ein oder mehrere, zum Beispiel 1, 2, 3, oder 4 Wasserstoffatome durch Fluoratome substituiert sein. Substituierte Cycloalkylreste können in beliebigen Positionen substituiert sein. Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. Dies gilt ebenso für substituierte Phenylreste in Gruppen wie zum Beispiel Phenylalkyl oder Phenyloxy. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind aromatische Ringverbindungen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über die 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für die Heteroarylreste wie zum Beispiel im Rest Heteroarylalkyl. Heteroaryl bedeutet zum Beispiel Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinyl.

Als Heteroarylreste gelten insbesondere 2- oder 3-Thienyl, 2- oder 3-Furyl, 1-, 2- oder 3- Pyrrolyl,1-, 2-, 4- oder 5-Imidazolyl, 1-, 3-, 4- oder 5-Pyrazolyl, 1,2,3-Triazol-1-, -4- oder -5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder -5-yl, 1,2,4-Oxadiazol-3- oder -5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder -5-yl, 2-, 3- oder 4-Pyridyl, 2-, 4-, 5- oder 6-Pyrimidinyl, 3- oder 4-Pyridazinyl, 2- oder 3-Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, -3- oder -4-pyridyl.

Besonders bevorzugt sind die Heteroaromaten 2- oder 3-Thienyl, 2- oder 3-Furyl, 1-, 2- oder 3- Pyrrolyl, 1-, 2-, 4- oder 5-Imidazolyl, 2-, 3-, 4-, 5-, 6-, 7- oder 8-Chinolyl, 1-, 3-, 4- oder 5-Pyrazolyl, 2-, 3- oder 4-Pyridyl, 2- oder 3-Pyrazinyl, 2-, 4-, 5- oder 6-Pyrimidinyl und 3- oder 4-Pyridazinyl.

Die Erfindung betrifft weiterhin ein Verfahren zur Herstellung der Verbindungen der Formel I oder deren Salze, dadurch gekennzeichnet, dass man Verbindungen der Formel II durch elektrophile aromatische Substitution zu Verbindungen der Formel I umsetzt, 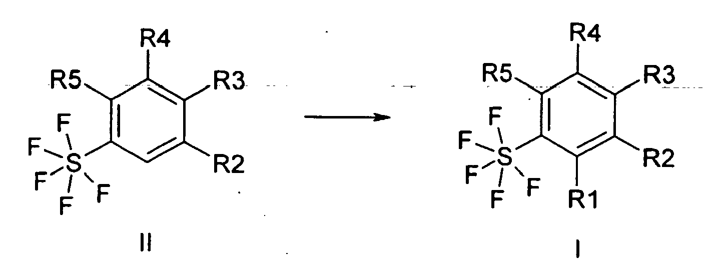 wobei R1 bis R5 die oben angegebene Bedeutung besitzen.

Bei der Herstellung der Verbindungen der Formel I geht man so vor, dass eine elektrophile aromatische Substitution durchgeführt wird, bevorzugt eine Halogenierung, Chlorsulfonierung oder Nitrierung.

In einer Ausführungsform wird halogeniert (R1 = Cl, Br oder I), wie in R.C. Larock, Comprehensive Organic Transformations: A Guide to Functional Group Preparations, VCH Publishers, New York, Weinheim, 1999, S.619-628 und in der dort zitierten Literatur beschrieben. Die Chlorierung erfolgt beispielsweise mit NCIS in einem inerten Lösungsmittel wie zum Beispiel Isopropanol, CHCl₃, CH₂Cl₂ oder EE bei einer Temperatur zwischen -30°C und 100°C, bevorzugt zwischen 40°C und dem Siedepunkt des Lösungsmittels.

In einer anderen Ausführungsform wird sulfoniert oder chlorsulfoniert (R1 = SO₂R6 mit R6 ist OH oder Cl), wie in March's Advanced Organic Chemistry 5th Edition 2001, S. 702-703 und in der dort zitierten Literatur beschrieben.

In einer anderen Ausführungsform wird nitriert (R1 = NO₂), wie beispielsweise in Houben-Weyl, Methoden der organischen Chemie, 4. Auflage, Organo-Stickstoff-Verbindungen IV, Teil 1, Georg Thieme Verlag Stuttgart 1992, S. 262-341 und in der dort zitierten Literatur beschrieben. Verbindungen der Formel II mit R3 = COOH werden beispielsweise mit einem Gemisch aus 90% HNO₃ und 96% H₂SO₄ bei einer Temperatur zwischen -40°C und 80°C, bevorzugt zwischen 0°C und 40°C nitriert.

Aus den Verbindungen der Formel mit R1 = NO₂ können die entsprechenden Aniline (R1 = NH₂) wie in R.C. Larock, Comprehensive Organic Transformations: A Guide to Functional Group Preparations, VCH Publishers, New York, Weinheim, 1999, 821-828 und der dort zitierten Literatur beschrieben hergestellt werden. Aus diesen Anilinen werden über die Diazoniumsalze nach dem Fachmann bekannter Methode, wie zum Beispiel in Houben-Weyl, Methoden der organischen Chemie, 4. Auflage, Organo-Stickstoff-Verbindungen I, Teil 2, Georg Thieme Verlag Stuttgart 1990, S. 1060-1136 sowie in den dort zitierten Literaturstellen beschrieben, die Verbindungen der Formel I mit weiteren Bedeutungen von R1 synthetisiert.

Die Ausgangsverbindungen der Formeln II sind käuflich erhältlich oder können nach analog zu in der Literatur beschriebenen und/oder dem Fachmann bekannten Verfahren hergestellt werden.

In den Ausgangsverbindungen können auch funktionelle Gruppen in geschützter Form oder in Form von Vorstufen vorliegen und dann in den nach dem oben beschriebenen Verfahren hergestellten Verbindungen der Formel I in die gewünschten Gruppen überführt werden. Entsprechende Schutzgruppentechniken sind dem Fachmann bekannt.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Beansprucht werden weiterhin die Verbindungen der Formel I und/oder deren Salze zur Verwendung als Syntheseintermediat, insbesondere zur Verwendung als Syntheseintermediat für die Herstellung von Medikamenten, Diagnostika, Flüssigkristallen, Polymeren, Pestiziden, Herbiziden, Fungiziden, Nematiziden, Parasitiziden, Insektiziden, Akariziden und Arthropodiziden.

Belspiele für die vielfältigen verwendungsmöglichkeiten von Pentafluorsulfanyl-Derivaten werden in den folgenden Veröffentlichungen beschrieben: WO9421606, WO03093228 (Insektizide, Akarizide); DE19711953, GB2276379 (Herbizide); DE10124480, DE10353658, Angew. Chem. 1999, 111, 2174, Angew. Chem. 2000, 112, 4384 (Flüssigkristalle); WO03097591, DE10353202 (Medikamente, Diagnostika); US5220070, US5302692 (Polymere); WO03093228, WO9625401 (Pestizide), GB2276381, GB2276380 (Fungizide), US5637607 (Nematizide), WO9947139 (Parasitizide), US6531501, WO9516676 (Arthropodizide).

Die Verbindungen der Formel I können in Form ihrer Salze isoliert werden. Diese werden nach den üblichen Methoden durch Umsetzung mit Säuren oder Basen erhalten. Als Säureadditionssalze kommen dabei beispielsweise Halogenide, insbesondere Hydrochloride, Hydrobromide, Lactate, Sulfate, Citrate, Tartrate, Acetate, Phosphate, Methylsulfonate, Benzolsulfonate, p-Toluolsulfonate, Adipinate, Fumarate, Gluconate, Glutamate, Glycerolphosphate, Maleinate, Benzoate, Oxalate und Pamoate und Trifluoracetate in Frage, im Fall der Herstellung von Wirkstoffen bevorzugt pharmazeutisch verträgliche Salze. Enthalten die Verbindungen eine Säuregruppe, können sie Salze mit Basen bilden, beispielsweise Alkalimetallsalze, vorzugsweise Natrium- oder Kaliumsalze, oder Ammoniumsalze, zum Beispiel als Salze mit Ammoniak oder organischen Aminen oder Aminosäuren. Sie können auch als Zwitterion vorliegen.

### Liste der Abkürzungen:

- DBU: 1,8-Diazabicyclo[5.4.0]undec-7-en 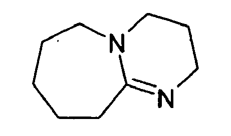
- DIP: Diisopropylether
- DIPEA: Diisopropylethylamin
- DME: 1,2-Dimethoxyethan
- DMF: N,N-Dimethylformamid
- EE: Ethylacetat (EtOAc)
- eq.: Äquivalent
- HEP: n-Heptan
- HOAc: Essigsäure
- MeOH: Methanol
- mp: Schmelzpunkt
- MTB: tert.-Butyl-methylether
- NCIS: N-Chlocsuccinimid
- dppf: 1,1 -Bis-(diphenylphosphino)-ferrocen
- RT: Raumtemperatur
- THF: Tetrahydrofuran

### Beispiel 1: 2-Methyl-5-nitro-4-pentafluorsulfanyl-benzoesäure

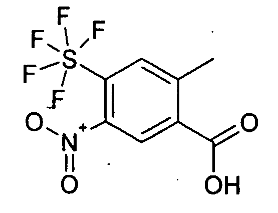

### a) 4-Aminophenyl-schwefelpentafluorid

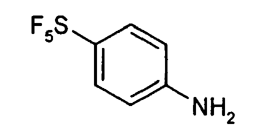

Eine Lösung von Zinn(II)chlorid (1465 g, 7,73 Mol) in konzentrierter (32-prozentiger) wässriger HCl-Lösung wurde unter Rühren auf 80 °C erwärmt und dann unter Eiskühlung innerhalb von 1 h in 8 Portionen 4-Nitrophenyl-schwefelpentafluorid (584 g, 2,344 Mol) eingetragen. Die Innentemperatur wurde hierbei unter 100 °C gehalten. Anschließend wurde das Gemisch 1,5 h bei 85 °C Innentemperatur gerührt und dann innerhalb einer weiteren Stunde auf 45 °C abkühlen gelassen. Ein Gemisch aus Eis (12 kg), NaOH (2 kg) und Dichlormethan (1,5 L) wurde vorbereitet und das Reaktionsgemisch unter starkem Rühren zugegeben. Die Phasen wurden getrennt, die wässrige Phase wurde 3 x mit je 1 L Dichlormethan extrahiert und die vereinigten organischen Phasen wurden über Na₂SO₄ getrocknet und im Vakuum eingedampft.

Man erhielt 510 g 4-Aminophenyl-schwefelpentafluorid als hellgelbes, kristallines Pulver, mp. 63-65 °C (Lit.: 57-59 °C).

### b) 4-Amino-3-brom-phenyl-schwefelpentafluorid

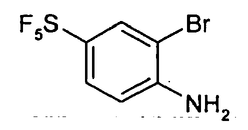

4-Aminophenyl-schwefelpentafluorid (510 g, 2,327 Mol) wurden in Dichlormethan (7 L) gelöst, die Lösung auf 5 °C abgekühlt und unter Rühren 1,3-Dibrom-5,5-dimethyl-imidazolidin-2,4-dion (326 g, 1,14 Mol) in mehreren Portionen unter Eiskühlung so eingetragen, dass die Innentemperatur bei 3-8 °C gehalten wurde (etwa 1 h). Anschließend wurde das Gemisch ohne äußere Kühlung 1 h rühren und auf Raumtemperatur aufwärmen gelassen. Das Gemisch wurde über ein Kieselgelbett (Volumen etwa 1 L) filtriert, mit Dichlormethan (5,5 L) nachgewaschen und das Filtrat im Vakuum eingedampft. Man erhielt etwa 700 g einer rotbraunen, kristallinen Masse, die bei 60 °C in n-Heptan (600 mL) gelöst wurde und danach im Kühlschrank bei 4 °C kristallisiert. Nach dem Absaugen erhielt man 590 g (85 %) 4-Amino-3-brom-phenyl-schwefelpentafluorid als bräunliche Kristall,
mp. 59-59,5 °C.

### c) 4-Amino-3-methyl-phenyl-schwefelpentafluorid

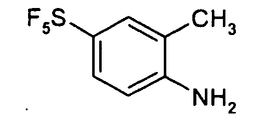

Ein Gemisch aus Cs₂CO₃ (794 g, 2,7 Mol), Dimethoxyethan (2 L), Wasser (300 mL) und Trimethylboroxin (50-prozentige Lösung in THF, 225 g, 0,9 Mol) wurde auf 70 °C erwärmt, PdCl₂(dppf) x CH₂Cl₂ (37 g, 45 mmol) zugegeben und eine Lösung von 4-Amino-3-brom-phenyl-schwefelpentafluorid (270 g, 0,9 Mol) in Dimethoxyethan (400 mL) innerhalb von 2 h unter Erhitzen des Reaktionsgemisches am Rückfluß zugetropft. Anschließend wurde weitere 3 h am Rückfluß erhitzt, dann auf Raumtemperatur abgekühlt, mit MTB (500 mL) verdünnt, über eine Kieselgelsäule (14 x 7 cm, 70-200 µm) filtriert und mit MTB (2500 mL) nachgewaschen. Das Filtrat wurde im Vakuum eingedampft. Man erhielt 490 g einer schwarzen, halbkristallinen Masse, die einer Wasserdampfdestillation unterzogen wurde. Insgesamt wurden 5,5 L Kondensat gesammelt, aus dem das Produkt sich bereits kristallin abscheidet. Das Kondensat wird 3 x mit MTB extrahiert, die vereinigten organischen Phasen über Na₂SO₄ getrocknet und im Vakuum eingedampft. Man erhielt 4-Amino-3-methyl-phenyl-schwefelpentafluorid (181 g, 76 %) als farblose Kristalle, mp. 65-66 °C.

### d) 4-Brom-3-methyl-phenyl-schwefelpentafluorid

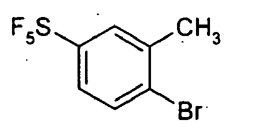

Ein Gemisch aus tert.-Butylnitrit (90-prozentig, 37 ml, 280 mmol), CuBr₂ (35.8 g, 160 mmol) in Acetonitril (260 mL) wurde bei 5 °C vorgelegt und unter Rühren und Eiskühlung eine Lösung von 4-Amino-3-methyl-phenyl-schwefelpentafluorid (30,9 g, 132,5 mmol) in MTB (140 mL) innerhalb 1 h bei 5-8 °C zugetropft. Dabei setzte nach etwa 2 min Stickstoff-Entwicklung ein. Anschließend wurde das Gemisch innerhalb 1 h unter Rühren auf Raumtemperatur erwärmen gelassen, ein Gemisch aus Eis (250 g), 26-prozentiger wässriger NH₃-Lösung (50 mL) und MTB (250 mL) zugesetzt und das Gemisch 10 min gerührt. Die Phasen wurden getrennt, die wässrige Phase 3 x mit MTB (je 150 mL) extrahiert und die vereinigten organischen Phasen einmal mit 400 mL Wasser geschüttelt. Nach Trocknen mit Na₂SO₄ und Eindampfen der organischen Phase erhielt man 39 g 4-Brom-3-methyl-phenyl-schwefelpentafluorid als rotbraunes Öl, das mit 8 Mol-% 4,5-Dibrom-3-methyl-phenyl-schwefelpentafluorid verunreinigt war, aber ohne weitere Reinigung weiterverwendet wurde. Ausbeute 89 %, bezogen auf eine Reinheit von 90 %.

### e) 4-Cyan-3-methyl-phenyl-schwefelpentafluorid

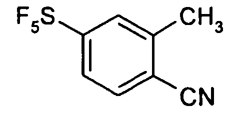

Ein Gemisch aus 4-Brom-3-methyl-phenyl-schwefelpentafluorid (136,4 g, Reinheit 80 %, 0,367 Mol), Zn(CN)₂ (72,8 g, 0,62 Mol) und Zn-Staub (7,2 g, 0,11 Mol) wurde in Dimethylacetamid (900 mL) und Wasser (40 mL) unter Stickstoff-Begasung vorgelegt, unter Rühren auf 125 °C erwärmt und PdCl₂(dppf) x CH₂Cl₂ (32,7 g, 40 mmol) zugesetzt. Nach einstündigem Rühren bei 125°C wurde nochmals PdCl₂(dppf) x CH₂Cl₂ (16,3 g, 20 mmol) und Zn-Staub (3,6 g, 55 mmol) zugegeben und weitere 2 h bei 125°C gerührt. Anschließend wurde auf Raumtemperatur abgekühlt, mit n-Heptan (400 mL) verdünnt und das Gemisch unter Zugabe von 5 N wässriger NH₄Cl-Lösung (250 mL) und Wasser (450 mL) 15 min lang stark gerührt. Das Gemisch wurde über eine Kieselgurschicht abgesaugt, die Phasen getrennt und die wässrige Phase 2 x mit n-Heptan (200 mL) extrahiert. Die vereinigten organischen Phasen wurden mit Wasser (450 mL) geschüttelt, über MgSO₄ getrocknet und im Vakuum eingedampft. Der erhaltene schwarze Rückstand wurde in 200 mL n-Heptan gelöst, filtriert und erneut im Vakuum eingedampft. Man erhielt 78 g einer dunkelbraunen Flüssigkeit, die durch Chromatographie an einer Kieselgelsäule (7 x 55 cm, 60-200 µm, n-Heptan/Dichlormethan 4:1 bis 3:2) gereinigt wurde. Als erste Fraktion erhielt man 6,5 g 4-Brom-3-methyl-phenyl-schwefelpentafluorid (Edukt) als gelbliche Flüssigkeit und anschließend 71,1 g (80 %) 4-Cyan-3-methyl-phenyl-schwefelpentafluorid als hellgelbes Öl.

### f) 2-Methyl-4-pentafluorsulfanyl-benzoesäure

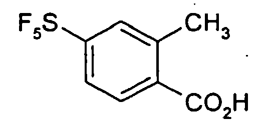

Ein Gemisch aus 4-Cyan-3-methyl-phenyl-schwefelpentafluorid (41,2 g, 169,4 g), NaOH (20,4 g, 510 mmol) und Wasser (60 mL) in Ethylenglykol (160 mL) wurde auf 130 °C erwärmt und 4 h bei dieser Temperatur gerührt. Anschließend wurde auf Raumtemperatur abgekühlt, mit MTB (150 mL) und Wasser (250 mL) verdünnt und das Gemisch abgesaugt. Die Phasen des Filtrats wurden getrennt und die wässrige Phase mit konzentrierter wässriger HCl-Lösung angesäuert und der ausfallende Feststoff abgesaugt. Man erhielt 41,1 g (93 %) 2-Methyl-4-pentafluorsulfanyl-benzoesäure als farblose Kristalle, mp. 138-139 °C.

### g) 2-Methyl-5-nitro-4-pentafluorsulfanyl-benzoesäure

6.0 g 2-Methyl-4-pentafluorsulfanyl-benzoesäure wurden in 60 ml einer 90% wässrigen HNO₃-Lösung gelöst und bei RT 6 ml einer 96% H₂SO₄ zugetropft. 28 h wurde bei RT stehen gelassen, anschließend auf 300 g Eis gegossen, 300 ml Wasser zugegeben, 1 h nachgerührt und dann das Produkt abfiltriert. An der Luft wurde getrocknet und man erhielt 6.5 g eines blassgelben Feststoffs, mp 218-220°C.
R_{f} (DIP/2%HOAc) = 0.27 MS(ES-): 306

### Beispiel 2: 3-Amino-4-chloro-5-pentafluorsulfanyl-benzoesäure-methylester

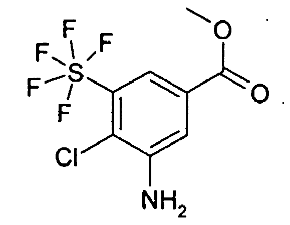

und 5-Amino-2-chloro-3- pentafluorsulfanyl-benzoesäure-methylester

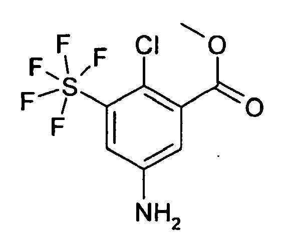

### a) 3-Pentafluorsulfanyl-benzoesäure

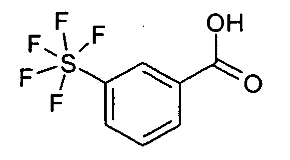

13.00 g (3-lodphenyl)schwefelpentafluorid (Tetrahedron 56, (2000) 3399) und 6.15 g Methyliodid wurden in 200 ml Diethylether (wasserfrei) gelöst und die Lösung zu 2.87 g Magnesium/ 20 ml Diethylether langsam zugetropft. Eine Stunde wurde im Rückfluss nachgerührt, dann auf -10°C gekühlt und das Reaktionsgemisch mit CO₂ unter Normaldruck begast. 16 Stunden lang wurde bei RT gerührt, anschließend das Reaktionsgemisch mit verdünnter wässeriger HCl-Lösung auf pH = 3-4 gestellt und 3 mal mit je 200 ml EE extrahiert. Über MgSO₄ wurde getrocknet und das Solvens im Vakuum entfernt. Man erhielt 7.20 g eines farblosen, amorphen Pulvers.
Rf (DIP/2%HOAc) = 0.51 MS (DCI): 249

### b) 3-Nitro-5- pentafluorsulfanyl-benzoesäure

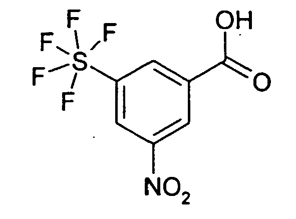

4.0 g 3-Pentafluorsulfanyl-benzoesäure wurden bei RT in 50 ml 100% HNO₃ gelöst und unter Eiskühlung 10 ml H₂SO₄ addiert. 6 Tage wurde bei RT gerührt, anschließend auf 200 g Eis gegossen, eine Stunde nachgerührt und schließlich das Produkt abgesaugt. Man erhielt 4.4 g hellgelber Kristalle, mp 140°C.
MS (ES-): 292

### c) 3-Nitro-5-pentafluorsulfanyl-benzoesäure-methylester

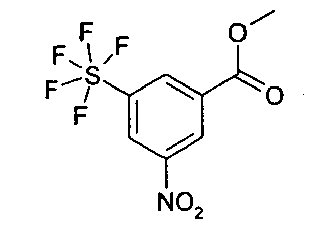

4.4 g 3-Nitro-5- pentafluorsulfanyl-benzoesäure wurden in 100 ml MeOH gelöst und bei RT 5.4 ml SOCl₂ zugetropft. 5 h wurde unter Rückfluss gekocht, die flüchtigen Bestandteile im Vakuum entfernt und 1 mal mit 100 ml Toluol co-evaporiert. Der Rückstand wurde an Kieselgel mit EE/HEP 1:8 chromatographiert und man erhielt 4.2 g eines farblosen Öls.
Rf (EE/HEP 1:8) = 0.18 MS (DCI): 308

### d) 3-Amino-5-pentafluorsulfanyl-benzoesäure-methylester

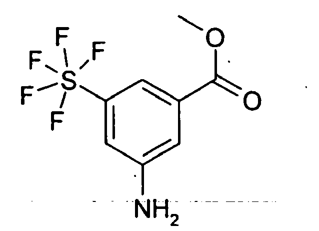

3.0 g 3-Nitro-5-pentafluorsulfanyl-benzoesäure-methylester wurden in 50 ml MeOH und 5 ml HOAc gelöst und 200 mg Pd/C (10%) zugegeben. 20 h wurde unter Normaldruck-Wasserstoffatmosphäre hydriert, anschließend weitere 2 Tage unter 6 bar Wasserstoff hydriert. Der Katalysator wurde abfiltriert, die Lösungsmittel im Vakuum entfernt und man erhielt 2.5 g eines amorphen Feststoffs.
Rf (DIP) = 0.48 MS (DCI): 278

### e) 3-Amino-4-chloro-5-pentafluorsulfanyl-benzoesäure-methylester und 5-Amino-2-chloro-3-pentafluorsulfanyl-benzoesäure-methylester

2.2 g 3-Amino-5-pentafluorsulfanyl-benzoesäure-methylester wurden in 20 ml Isopropanol gelöst und bei 60°C 1.1 g NCIS addiert. 2 h wurde unter Rückfluss gekocht, anschließend ließ man die Lösung auf RT abkühlen. Dann wurden 10 ml einer gesättigten wässrigen Na₂SO₃-Lösung und 100 ml einer gesättigten wässrigen Na₂CO₃-Lösung addiert und 3 mal mit je 150 ml EE extrahiert. Über MgSO₄ wurde getrocknet, das Solvens im Vakuum entfernt und der Rückstand an Kieselgel mit EE/HEP 1:6 chromatographiert. Man erhielt 508 mg 3-Amino-4-chloro-5-pentafluorsulfanyl-benzoesäure-methylester sowie 94 mg 5-Amino-2-chloro-3-pentafluorsulfanyl-benzoesäure-methylester neben 1.39 g 3-Amino-2-chloro-5-pentafluorsulfanyl-benzoesäure-methylester; jeweils als farblose Öle.
Rf (EE/HEP 1:6) = 0.26: 3-Amino-2-chloro-5-pentafluorsulfanyl-benzoesäure-methylester
Rf (EE/HEP 1:6) = 0.15: 3-Amino-4-chloro-5-pentafluorsulfanyl-benzoesäure-methylester
Rf (EE/HEP 1:6) = 0.26: 5-Amino-2-chloro-3-pentafluorsulfanyl-benzoesäure-methylester
MS (ES+): jeweils 352 (M+CH₃C=N)

### Beispiel 3: 2-Chloro-3-pentafluorsulfanyl-anilin und 4-Chloro-3-pentafluorsulfanyl-anilin

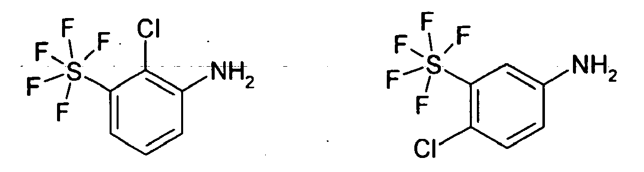

8.00 g 3-pentafluorsulfanyl-anilin (Tetrahedron 56, (2000) 3399) wurden in 200 ml Isopropanol gelöst und bei 60°C 4.87 g NCIS portionsweise zugegeben (innerhalb 30 Minuten). Weitere 20 Minuten wurde bei 60°C gerührt, anschließend 2 h unter Rückfluss gekocht. Man ließ das Reaktionsgemisch auf RT abkühlen und entfernte die Hälfte des Solvens im Vakuum. Dann wurden 300 ml einer halbgesättigten wässrigen NaHCO₃-Lösung und 50 ml einer gesättigten wässrigen Na₂SO₃-Lösung zugegeben und 3 mal mit je 100 ml CH₂Cl₂ extrahiert. Über Mg SO₄. wurde getrocknet, das Solvens im Vakuum entfernt und der Rückstand an Kieselgel mit EE/HEP 1:4 chromatographiert. Man erhielt 2.02 g 2-Chloro-3-pentafluorsulfanyl-anilin und 1.10 g 4-Chloro-3-pentafluorsulfanyl-anilin neben 2.73 g 2-Chloro-5-pentafluorsulfanyl-anilin.
Rf (EE/HEP 1:4) = 0.31: 2-Chloro-5-pentafluorsulfanyl-anilin
Rf (EE/HEP 1:44) = 0.18: 2-Chloro-3-pentafluorsulfanyl-anilin
Rf (EE/HEP 1:4) = 0.11: 4-Chloro-3-pentafluorsulfanyl-anilin
MS (DCI): jeweils 253

### Beispiel 4: 2-(4-Nitro-3-pentafluorsulfanyl-phenyl)-isoindol-1,3-dion und 2-(2-Nitro-5-pentafluorsulfanyl-phenyl)-isoindol-1,3-dion

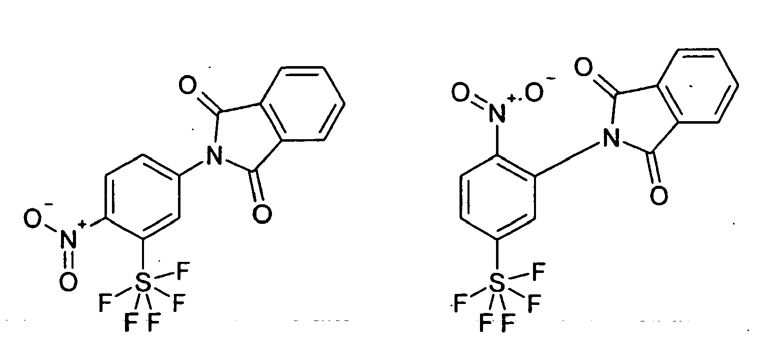

### a) 2-(3-Pentafluorsulfanyl-phenyl)-isoindol-1,3-dion:

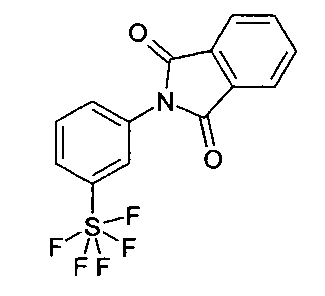

15 g (68.44 mmol) 3-Pentafluorsulfanyl-phenylamin wurden mit 10.14 g (68.44 mmol) Phthalsäureanhydrid in 40 ml Essigsäure suspendiert und 2 h unter Rückfluß gekocht. Die abgekühlte Reaktionsmischung wurde mit 400 ml Wasser versetzt, 30 min. im Ultraschallbad behandelt und filtriert. Der Rückstand wurde mit Wasser und anschließend mit wenig Ethanol gewaschen und im Vakuum getrocknet. Man erhielt 2-(3-Pentafluorsulfanyl-phenyl)-isoindol-1,3-dion mit dem Schmelzpunkt 188-190°C.

### b) 2-(4-Nitro-3-pentafluorsulfanyl-phenyl)-isoindol-1,3-dion und 2-(2-Nitro-5-pentafluorsulfanyl-phenyl)-isoindol-1,3-dion,

1 g (2.863 mmol) 2-(3-Pentafluorsulfanyl-phenyl)-isoindol-1,3-dion wurde bei 0°C in 3.29 ml konzentrierter Salpetersäure gelöst, und die Mischung wurde für 2 h bei 0°C gerührt. Danach ließ man über Nacht bei Raumtemperatur stehen. Die Reaktionslösung wurde auf 50 g Eiswasser gegeben und die Mischung 1 h gerührt; danach wurde der Niederschlag abgesaugt, mit Wasser gewaschen, getrocknet und über Kieselgel mit Toluol als Laufmittel chromatographisch gereinigt. Man erhielt 2-(4-Nitro-3-pentafluorsulfanyl-phenyl)-isoindol-1,3-dion mit dem Schmelzpunkt 200-203°C
und 2-(2-Nitro-5-pentafluorsulfanyl-phenyl)-isoindol-1,3-dion mit dem Schmelzpunkt 175-177°C im Verhältnis 1:2.

### Beispiel 5: 2-(4-Amino-3-pentafluorsulfanyl-phenyl)-isoindol-1,3-dion (nicht erfindungsgemäß)

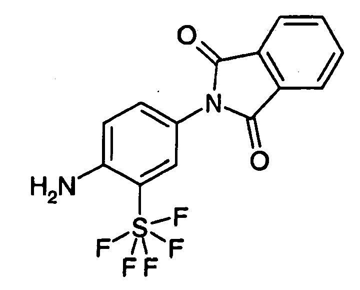

1.94 g (4.92 mmol) 2-(4-Nitro-3-pentafluorsulfanyl-phenyl)-isoindol-1,3-dion (hergestellt in Beispiel 4) wurden in 20 ml Methanol gelöst, mit 53 mg Palladium 10% ig auf Aktivkohle versetzt und bei Raumtemperatur bei einem Wasserstoffdruck von 5 bar hydriert. Nach beendeter Reaktion wurde vom Katalysator abfiltriert und das Filtrat eingeengt. Der Rückstand wurde in einer Mischung aus Dichlormethan und n-Heptan verrührt, abgesaugt und im Vakuum getrocknet. Man erhielt 2-(4-Amino-3-pentafluorsulfanyl-phenyl)-isoindol-1,3-dion mit dem Schmelzpunkt 176-178°C.

Wurde die oben beschriebene Reaktion vorzeitig abgebrochen, so wurde 2-(4-Hydroxyamino-3-pentafluorsulfanyl-phenyl)-isoindol-1,3-dion 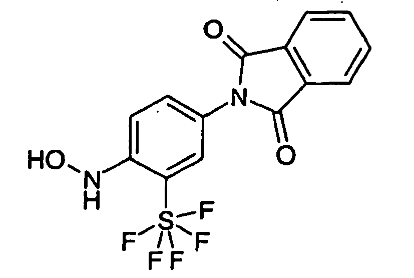 mit einem Schmelzpunkt (unter Zersetzung) von 171-173°C erhalten.

### Beispiel 6: 4-(1,3-Dioxo-1,3-dihydro-isoindol-2-yl)-2-pentafluorsulfanyl-benzonitril (nicht erfindungsgemäß):

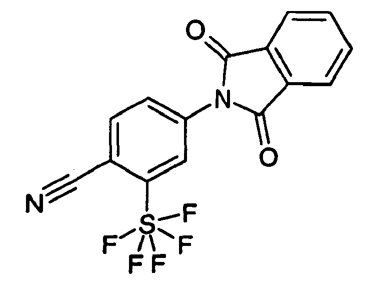

Einer Lösung von 1 g (2.74 mmol) 2-(4-Amino-3-pentafluorsulfanyl-phenyl)-isoindol-1,3-dion (hergestellt in Beispiel 5) in Essigsäure wurden bei 0°C langsam 0.46 ml (8.24 mmol) halbkonzentrierte Schwefelsäure zugetropft. Die Mischung wurde 10 min. bei 0°C gerührt; danach wurde unter Rühren langsam eine Lösung von 189.4 mg Natriumnitrit in 2 ml Wasser zugetropft, und die erhaltene Lösung wurde 30 min. bei 0°C gerührt. Diese Lösung wurde schließlich in eine auf 0°C gekühlte Lösung von 246 mg (2.74 mmol) Kupfer-I-cyanid und 536 mg (8.23 mmol) Kaliumcyanid in 5 ml Wasser zugetropft. Die Reaktionsmischung wurde 30 min. bei 0°C und danach noch 3 h bei Raumtemperatur gerührt. Nach Ende der Reaktion wurde die Mischung auf Wasser gegeben und die wässrige Phase zweimal mit Essigsäureethylester ausgeschüttelt. Die organische Phase wurde über Magnesiumsulfat getrocknet, filtriert, das Filtrat eingeengt und der Rückstand über Kieselgel zuerst mit Toluol und dann mit Toluol/Essigsäureethylester 20/1 chromatographisch gereinigt. Man erhielt 4-(1,3-Dioxo-1,3-dihydro-isoindol-2-yl)-2-pentafluorsulfanyl-benzonitril. ¹H NMR (500 MHz; d₆-dmso): δ [ppm] = 8.4 (m, 2H); 8.1-7.95 (m, 5H).

### Beispiel 7: 4-Amino-2-pentafluorsulfanyl-benzonitril und N-(4-Cyano-3-pentafluorsulfanyl-phenyl)-phthalsäureamid ethylester (nicht erfindungsgemäß)

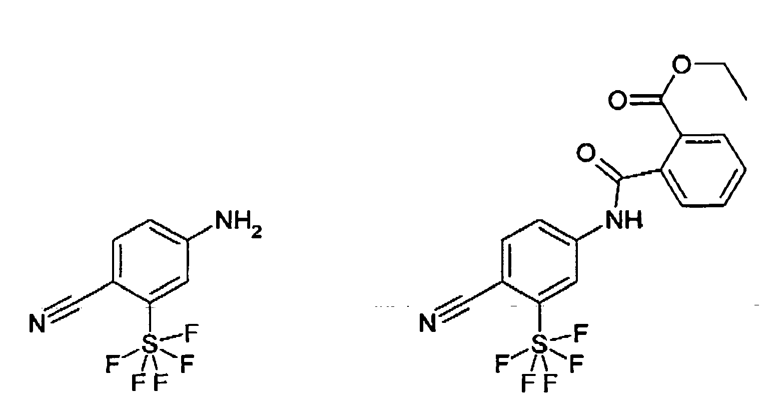

610 mg (1.63 mmol) 4-(1,3-Dioxo-1,3-dihydro-isoindol-2-yl)-2-pentafluorsulfanyl-benzonitril (hergestellt in Beipspiel 6) wurden in 30 ml Ethanol gelöst und mit 100 mg (1.956 mmol) Hydrazinhydrat (100 %) versetzt. Die Mischung wurde über Nacht bei Raumtemperatur gerührt. Danach wurde die Reaktionsmischung im Vakuum eingeengt und der Rückstand chromatographisch (präparative HPLC; Purospher STAR RP-18e (10 µm); Laufmittel: Acetonitril/Wasser (0-5% Trifluoressigsäure) 5/95 → 95/5 [45 min.]) gereinigt. Man erhielt 4-Amino-2-pentafluorsulfanyl-benzonitril (¹H NMR (500 MHz; d₆-dmso) δ [ppm] = 7.65 (s, 1H); 7.2 (s, 1H; 6.8 (m, 3H)) und N-(4-Cyano-3-pentafluorsulfanyl-phenyl)-phthalsäureamid ethylester (¹H NMR (500 MHz; d₆-dmso) δ [ppm]=11.3 (s, 1H); 8.6 (s, 1H); 8.2 (d, 1H); 8.1 (d, 1H); 7.95 (d, 1H); 7.75 (m, 1H); 7.7 (m, 2H); 4.2 (q, 2H); 1.15 (t, 3H)).

### Beispiel 8: N-(4-Nitro-3-pentafluorsulfanyl-phenyl)-acetamid und N-(2,4-Dinitro-5-pentafluorsulfanyl-phenyl)-acetamid

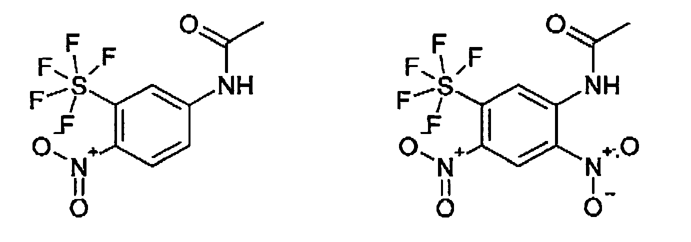

1.0 g N-(3-Pentafluorsulfanyl-phenyl)-acetamid (Darstellung wie in Tetrahedron 56, (2000) 3399) wurden bei 0-3°C in 10 ml 90% HNO₃ portionsweise gelöst. 15 Minuten wurde bei 0°C gerührt, dann auf 100g Eis gegossen und 3 mal mit je 100 ml EE extrahiert. Über MgSO₄ wurde getrocknet, das Solvens im Vakuum entfernt und der Rückstand an Kieselgel mit DIP chromatographiert. Man erhielt 195 mg N-(4-Nitro-3-pentafluorsulfanyl-phenyl)-acetamid und 280 mg N-(2,4-Dinitro-5-pentafluoro-sulfanyl-phenyl)-acetamid neben 645 mg N-(2-Nitro-5-pentafluorsulfanyl-phenyl)-acetamid.
Rf (DIP) = 0.41: N-(2-Nitro-5-pentafluorsulfanyl-phenyl)-acetamid MS (EI): 306
Rf (DIP) = 0.18: N-(2,4-Dinitro-5-pentafluorsulfanyl-phenyl)-acetamid MS (EI): 351
Rf (DIP) = 0.11: N-(4-Nitro-3-pentafluorsulfanyl-phenyl)-acetamid MS (EI): 306

### Beispiel 9: N-(4-Nitro-3-pentafluorsulfanyl-phenyl)-acetamid

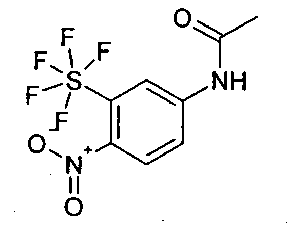

20.00 g N-(3-Pentafluorsulfanyl-phenyl)-acetamid (Darstellung wie in Tetrahedron 56, (2000) 3399) wurden bei -35°C bis -40°C in 100 ml 90% HNO₃ portionsweise gelöst. 15 Minuten wurde bei -40°C gerührt, dann auf 1kg Eis gegossen und 1 h bei RT gerührt. Dann wurde das Produkt abfiltriert, mit Wasser gewaschen und im Vakuum getrocknet. Chromatographie an Kieselgel mit DIP lieferte 3.61 g N-(4-Nitro-3-pentafluorsulfanyl-phenyl)-acetamid neben 17.00 g N-(2-Nitro-5-pentafluoro-sulfanyl-phenyl)-acetamid.
Rf (DIP) = 0.41: N-(2-Nitro-5-pentafluorsulfanyl-phenyl)-acetamid MS (EI): 306
Rf (DIP) = 0.11: N-(4-Nitro-3-pentafluorsulfanyl-phenyl)-acetamid MS (EI): 306

### Beispiel 10: 1,3-Dibrom-2-methoxy-4-nitro-5-pentafluorsulfanyl-benzol

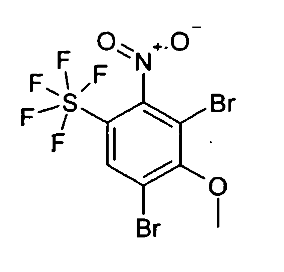

### a) 4-Pentafluorsulfanyl-phenol

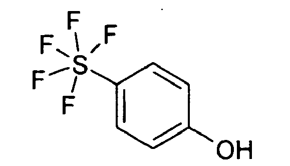

40.00 g 4-Pentafluorsulfanyl-anilin wurden in 500 ml einer 35% wässrigen H₂SO₄-Lösung suspendiert und bei 0°C eine Lösung von 13.85 g NaNO₂ in 30 ml Wasser über einen Zeitraum von 10 Minuten zugetropft. Anschließend wurde 35 Minuten bei 0°C nachgerührt, dann eine 0°C kalte Lösung von 171.10 g Cu(NO₃)₂ in 200 ml Wasser zugegossen und direkt danach 26.11 g Cu₂O portionsweise addiert. 2 Stunden wird bei RT nachgerührt, dann 3 mal mit je 200 ml CH₂Cl2₂ extrahiert. Über MgSO₄ wurde getrocknet und das Solvens im Vakuum entfernt. Man erhielt 38.00 g eines blassgelben Öls, das ohne Reinigung weiter umgesetzt wurde.

### b) 4-Methoxy-pentafluorsulfanyl-benzol

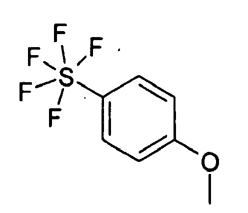

5.00 g 4-Pentafluorsulfanyl-phenol wurden in 50.00 g Dimethylcarbonat gelöst und 3.46 g DBU zugegeben. 10 Stunden wurde unter Rückfluss gekocht, dann ließ man abkühlen und verdünnte mit 200 ml EE. Anschließend wurde 2 mal mit je 100 ml einer 5% wässrigen HCl-Lösung, dann mit 100 ml einer 5% wässrigen NaOH-Lösung gewaschen. Über MgSO₄ wurde getrocknet und das Solvens im Vakuum entfernt. Chromatographie an Kieselgel mit DIP/HEP 1:1 lieferte 2.2 g eines farblosen Öls.
Rf (DIP/HEP 1:1) = 0.52

### c) 2,6-Dibromo-4-pentafluorsulfanyl-phenol

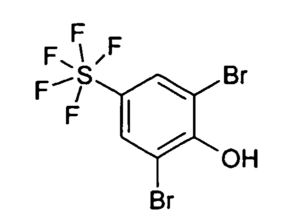

3.34 g 4-Methoxy-pentafluorsulfanyl-benzol wurden in 200 ml CHCl₃ gelöst und 0.46 g FeBr₂ zugegeben. Bei RT wurden dann 6.84 g Brom zugetropft und bei RT 4 Tage gerührt. Anschließend wurden weitere 400 mg FeBr₂ zugegeben und weitere 23 Stunden bei RT gerührt. Dann wurde das Reaktionsgemisch vorsichtig auf 100 ml einer gesättigten wässrigen Na₂SO₃-Lösung gegossen und 3 mal mit je 50 ml CH₂Cl₂ extrahiert. Über MgSO₄ wurde getrocknet und das Solvens im Vakuum entfernt. Chromatographie an Kieselgel mit DIP
lieferte 3.00 g eines amorphen Feststoffs.
R_{f} (DIP) = 0.22

### d) 1,3-Dibromo-2-methoxy-5-pentafluorsulfanyl-benzol

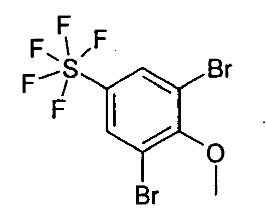

450 mg 2,6-Dibromo-4-pentafluorsulfanyl-phenol, 329 mg K₂CO₃ sowie 186 mg CH₃l wurden in 5 ml wasserfreiem DMF 24 Stunden bei RT gerührt. Anschließend wird das Reaktionsgemisch auf 100 ml EE gegossen und 3 mal mit je 30 ml Wasser gewaschen. Über MgSO₄ wurde getrocknet und das Solvens im Vakuum entfernt und man erhielt 500 mg eines farblosen Öls.
Rf (DIP/HEP 1:1) = 0.51 MS (EI): 392

### e) 1,3-Dibrom-2-methoxy-4-nitro-5-pentafluorsulfanyl-benzol

630 mg 1,3-Dibromo-2-methoxy-5-pentafluorsulfanyl-benzol wurden in 2 ml einer 90% wässrigen HNO₃-Lösung bei 0°C 1 Stunde lang gerührt. Anschließend wurde 20 Minuten lang bei RT gerührt und dann auf 50 g Eis gegossen. Mit einer wässrigen Na₂CO₃-Lösung wurde auf pH=6 gestellt und drei mal mit je 50 ml EE extrahiert. Über Na₂SO₄ wurde getrocknet und das Solvens im Vakuum entfernt. Chromatographie an Kieselgel mit DIP/HEP 1:3 lieferte 260 mg eines blassgelben Öls.
Rf (DIP/HEP 1:3) = 0.40

### Beispiet 11: 1-Brom-3-chlor-2-methoxy-4-nitro-5-pentafluorsulfanyl-benzol und 3-Brom-1-chlor-2-methoxy-4-nitro-5-pentafluorsulfanyl-benzol

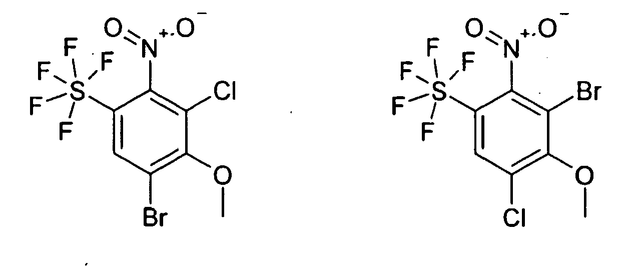

### a) 2-Chlor-4-pentafluorsulfanyl-phenol

5.00 g 4-Pentafluorsulfanyl-phenol (hergestellt in Beispiel 11a) wurden in 100ml Essigsäure gelöst und bei 0°C 10 Minuten lang ein Chlorgasstrom durchgeleitet. Dabei erwärmt sich die Lösung auf 30°C und wird anschließend noch 90 Minuten bei RT gerührt. Mit Argon wird das Chlor aus der Lösung ausgetrieben und anschließend das Solvens im Vakuum entfernt. Man erhält 5.50 g eines blassgelben Öls.
Rf (DIP) = 0.23

### b) 2-Chlor-1-methoxy-4-pentafluorsulfanyl-benzol

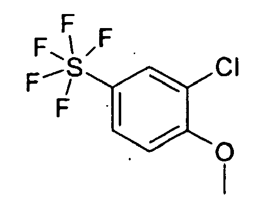

5,50 g 2-Chlor-4-pentafluorsulfanyl-phenol, 7.89 g K₂COO₃ sowie 4.05 g CH₃l wurden in 30 ml wasserfreiem DMF 2 Stunden bei RT gerührt und 2 Tage bei RT stehen gelassen. Dann wurde mit 300 ml EE verdünnt und 3 mal mit je 100 ml Wasser gewaschen. Über Na₂SO₄ wurde getrocknet und das Solvens im Vakuum entfernt und man erhielt 5.40 g eines blassgelben Öls.
Rf (DIP) = 0.68

### c) 2-Bromo-6-chloro-4-pentafluorsulfanyl-phenol

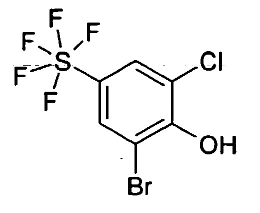

5.30 g 2-Chlor-1-methoxy-4-pentafluorsulfanyl-benzol wurden in 150 ml CHCl₃ gelöst und mit 4.73 g Brom sowie 638 mg FeBr₂ versetzt. 18 Stunden wurde bei RT gerührt, dann mit weiteren 200 mg FeBr₂ versetzt, 6 Stunden bei RT gerührt und dann mit weiteren 300 mg FeBr₂ versetzt, 2 Stunden bei RT gerührt und 18 Stunden bei RT stehen gelassen. Dann wurde das Reaktionsgemisch auf 300 ml einer gesättigten wässrigen Na₂SO₃-Lösung gegossen und mit 300 ml CH₂Cl₂ extrahiert. Die organische Phase wurde dann mit 100 ml Wasser gewaschen, über Na₂SO₄ getrocknet und das Solvens im Vakuum entfernt. Man erhielt 4.20 g eines farblosen Öls, das ohne Reinigung weiter umgesetzt wurde.

### d) 1-Brom-3-chlor-2-methoxy-5-pentafluorsulfanyl-benzol

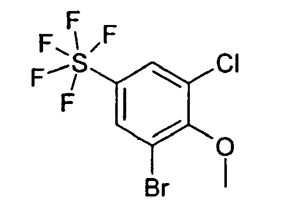

4.20 g 2-Bromo-6-chloro-4-pentafluorsulfanyl-phenol wurden zusammen mit 3.48 g K₂CO₃ und 2.68 g CH₃l in 50 ml wasserfreiem DMF 24 Stunden bei RT gerührt. Dann wurde das Solvens im Vakuum entfernt und anschließend mit je 100 ml Wasser und EE aufgenommen. Man ließ die Phasen trennen und extrahierte dann noch 2 mal mit je 100 ml EE. Über Na₂SO₄ wurde getrocknet und das Solvens im Vakuum entfernt. Chromatographie an Kieselgel mit DIP/HEP 1:1 lieferte 3.44 g einer farblosen viskosen Flüssigkeit.
Rf (DIP/HEP 1:1) = 0.53 MS (EI): 346

### e) 1-Brom-3-chlor-2-methoxy-4-nitro-5-pentafluorsulfanyl-benzol und 3-Brom-1-chlor-2-methoxy-4-nitro-5-pentafluorsulfanyl-benzol

3.40 g 1-Brom-3-chlor-2-methoxy-5-pentafluorsulfanyl-benzol wurden bei 0°C bis 5°C zu 40 ml einer 90% wässrigen HNO₃-Lösung zugetropft. 60 Minuten wurde bei 0°C gerührt, dann wurde 90 Minuten bei RT gerührt. Anschließend wurde das Reaktionsgemisch auf 200 g Eis gegossen und 3 mal mit je 200 ml EE extrahiert. Über Na₂SO₄ wurde getrocknet und das Solvens im Vakuum entfernt. Chromatographie an Kieselgel mit DIP/HEP 1:3 lieferte 2.00 g eines blassgelben Öls.
MS (EI): 391

### Beispiel 12: 2-Chlor-4-nitro-5-pentaftuorsulfanyl-anilin (nicht erfindungsgemäß)

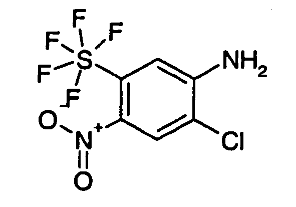

a) 2.60 g 2-Chloro-5-pentafluorsulfanyl-anilin (Beispiel 3) wurden bei 0°C zu 30 ml 100% HNO3 zugetropft. 1 Stunde wurde bei 0°C gerührt, dann auf 100 g Eis gegossen und mit gesättigter wässriger NaHCO₃-Lösung auf pH=7 gestellt. Dann wurde 3 mal mit je 100 ml EE extrahiert, anschließend über MgSO₄ getrocknet. Das Solvens wurde im Vakuum entfernt und man erhielt 2.50 g eines blassgelben Öls.
Rf (EE) = 0.13

## Patentansprüche

1. Verbindungen der Formel I 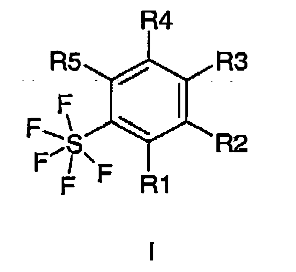 worin bedeuten
R1 Cl, Br, I, -SO₂R6 oder NO₂; R6 OH, F, Cl, Br, I oder Alkyl mit 1, 2, 3 oder 4 C-Atomen;
R2 und R4 unabhängig voneinander Wasserstoff, F, Cl, Br, I, -CN, -NR9R10, -OR11, SR12, COR13, -(SOᵣ)ₛ-(CH₂)ₜ-(CF₂)ᵤ-CF₃, Alkyl mit 1, 2, 3, 4, 5 oder 6 C- Atomen, Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Alkyl mit 1, 2, 3 oder 4 C-Atomen, -(CH₂)ᵥ -(CF₂)_{w}-CF₃, Alkylcarbonyl mit 1, 2, 3 oder 4 C- Atomen, Alkylsulfonyl mit 1, 2, 3 oder 4 C-Atomen;
oder
R9 und R10 bilden zusammen mit dem sie tragenden Stickstoffatom einen Heterocyclus der Formel IIIa-. 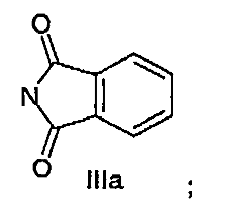
R11 und R12 unabhängig voneinander Wasserstoff, Alkyl mit 1, 2, 3 oder 4 C-Atomen, -(CH₂)ᵥ-(CF₂)_{w-}CF₃, Alkylcarbonyl mit 1, 2, 3 oder 4 C-Atomen, Alkylsulfonyl mit 1, 2, 3 oder 4 C-Atomen;
R13 OH, Alkyl mit 1, 2, 3, 4, 5 oder 6 C-Atomen oder Alkoxy mit 1, 2, 3, 4, 5 oder 6 Atomen;
s Null;
t und u unabhängig voneinander Null oder 1;
v und w unabhängig voneinander Null oder 1;
wobei mindestens einer der Reste R2 und R4 nicht Wasserstoff, Cl, CN, COR13 oder -(SOᵣ)ₛ-(CH₂)ₜ-(CF₂)ᵤ-CF₃ ist;
R3 Wasserstoff, F, Cl, Br, I, -CN, -NO₂, -COR14, -SO₂CH₃, Alkyl mit 1, 2, 3, 4, 5 oder 6 C-Atomen, Alkoxy mit 1, 2, 3 oder 4 C-Atomen, -Oₓ-(CH₂)_{y}-CF₃,
R14 OH, Alkyl mit 1, 2, 3, 4, 5 oder 6 C-Atomen, Alkoxy mit 1, 2, 3, 4, 5 oder 6 C-Atomen oder -Oₐₐ-(CH₂)_{bb}-CF₃;
x Null oder 1;
y Null, 1, 2 oder 3;
aa Null oder 1;
bb Null, 1, 2 oder 3;
R5 Wasserstoff oder F;
sowie deren Salze;
wobei Verbindungen der Formel I ausgeschlossen sind, in denen R2 und R4 Cl und R3 F oder Cl bedeuten,
wobei Verbindungen der Formel I ausgeschlossen sind, in denen einer der Substituenten R2 und R4 Cl und der andere der Substituenten R4 und R2 CN und R3 Cl bedeuten und
wobei Verbindungen der Formel I ausgeschlossen sind, in denen R1 NO₂ und die anderen Substituenten Wasserstoff bedeuten.

2. Verfahren zur Herstellung der Verbindungen der Formel I oder deren Salze, **dadurch gekennzeichnet, dass** man Verbindungen der Formel II durch elektrophile aromatische Substitution zu Verbindungen der Formel I umsetzt, 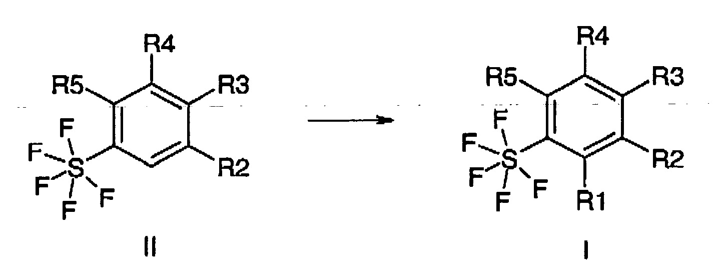 wobei R1 bis R5 die in Anspruch 1 angegebene Bedeutung besitzen.

3. Verbindung der Formel I und/oder deren Salze nach Anspruch 1 zur Verwendung als Syntheseintermediat.

4. Verbindung der Formel I und/oder deren Salze nach Anspruch 1 zur Verwendung als Syntheseintermediat für die Herstellung von Medikamenten, Diagnostika, Flüssigkristallen, Polymeren, Pestiziden, Herbiziden, Fungiziden, Nematiziden, Parasitiziden, Insektiziden, Akariziden und Arthropodiziden.

## Claims

1. A compound of the formula I 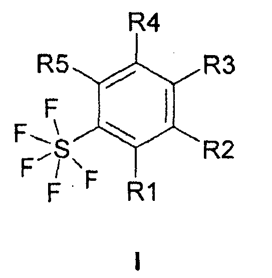 where
R1 is Cl, Br, I, -SO₂R6 or NO₂; R6 is OH, F, Cl, Br, I or alkyl having 1, 2, 3 or 4 carbon atoms;
R2 and R4 are each independently hydrogen, F, Cl, Br, I, -CN, -NR9R10, -OR11, -SR12, COR13, - (SOᵣ)ₛ-(CH₂)ₜ-(CF₂)ₜ-CF₃, alkyl having 1, 2, 3, 4, 5 or 6 carbon atoms, cycloalkyl having 3, 4, 5, 6, 7 or 8 carbon atoms, in which 1, 2, 3 or 4 hydrogen atoms may be replaced by fluorine atoms;
R9 and R10 are each independently alkyl having 1, 2, 3 or 4 carbon atoms, - (CH₂)ᵥ- (CF₂)_{w}-CF₃, alkylcarbonyl having 1, 2, 3 or 4 carbon atoms, alkylsulfonyl having 1, 2, 3 or 4 carbon atoms;
or
R9 and R10, together with the nitrogen atom bearing them, form a heterocycle of the formula IIIa: 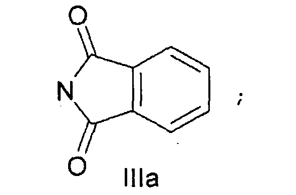
R11 and R12 are each independently hydrogen, alkyl having 1, 2, 3 or 4 carbon atoms, - (CH₂)ᵥ- (CF₂)_{w}-CF₃, alkylcarbonyl having 1, 2, 3 or 4 carbon atoms, alkylsulfonyl having 1, 2, 3 or 4 carbon atoms;
R13 is OH, alkyl having 1, 2, 3, 4, 5 or 6 carbon atoms or alkoxy having 1, 2, 3, 4, 5 or 6 carbon atoms;
s is zero;
t and u are each independently zero or 1;
v and w are each independently zero or 1;
where at least one of the R2 and R4 radicals is not hydrogen, Cl, CN, COR13 or - (SOᵣ)ₛ-(CH₂)ₜ-(CF₂)ᵤ-CF₃;
R3 is hydrogen, F, Cl, Br, I, -CN, -NO₂, -COR14, -SO₂CH₃, alkyl having 1, 2, 3, 4, 5 or 6 carbon atoms, alkoxy having 1, 2, 3 or 4 carbon atoms, -Oₓ- (CH₂)_{y}-CF₃,
R14 is OH, alkyl having 1, 2, 3, 4, 5 or 6 carbon atoms, alkoxy having 1, 2, 3, 4, 5 or 6 carbon atoms or -Oₐₐ-(CH₂)_{bb}-CF₃ ;
x is zero or 1;
y is zero, 1, 2 or 3;
aa is zero or 1;
bb is zero, 1, 2 or 3;
R5 is hydrogen or F;
and salts thereof;
excluding compounds of the formula I in which R2 and R4 are each Cl and R3 is F or Cl,
excluding compounds of the formula I in which one of the R2 and R4 substituents is Cl and the other of the R2 and R4 substituents is CN and R3 is Cl and
excluding compounds of the formula I in which R1 is NO₂ and the other substituents are each hydrogen.

2. A process for preparing compounds of the formula I or salts thereof, which comprises converting compounds of the formula II by electrophilic aromatic substitution to compounds of the formula I 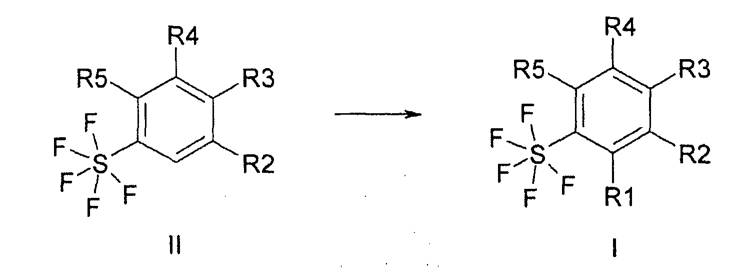 where R1 to R5 are each as defined in claim 1.

3. A compound of the formula I and/or salts thereof as claimed in claim .1 for use as a synthetic intermediate.

4. A compound of the formula I and/or salts thereof as claimed in claim 1 for use as a synthetic intermediate for the preparation of medicaments, diagnostic aids, liquid crystals, polymers, pesticides, herbicides, fungicides, nematicides, parasiticides, insecticides, acaricides and arthropodicides.

## Revendications

1. Composés de formule I 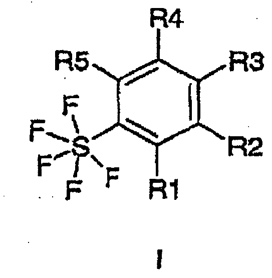 dans laquelle
R1 signifie Cl, Br, I, -SO₂R6 ou NO₂ ; R6 signifie OH, F, Cl, Br, I ou alkyle comprenant 1, 2, 3 ou 4 atomes de carbone ;
R2 et R4 signifient indépendamment l'un de l'autre hydrogène, F, Cl, Br, I, -CN, -NR9R10, -OR11, -SR12, COR13, -(SOᵣ)ₛ-(CH₂)ₜ-(CF₂)ₜCF₃, alkyle comprenant 1, 2, 3, 4, 5 ou 6 atomes de carbone, cycloalkyle comprenant 3, 4, 5, 6, 7 ou 8 atomes de carbone, où 1, 2, 3 ou 4 atomes d'hydrogène peuvent être remplacés par des atomes de fluor ;
R9 et R10 signifient indépendamment l'un de l'autre alkyle comprenant 1, 2, 3 ou 4 atomes de carbone, - (CH₂)ᵥ-(CF₂)_{w}-CF₃, alkylcarbonyle comprenant 1, 2, 3 ou 4 atomes de carbone, alkylsulfonyle comprenant 1, 2, 3 ou 4 atomes de carbone ; ou
R9 et R10 forment ensemble avec l'atome d'azote qui les porte un hétérocycle de formule IIIa : 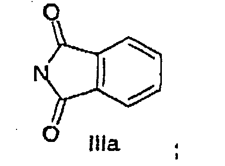
R11 et R12 signifient indépendamment l'un de l'autre hydrogène, alkyle comprenant 1, 2, 3 ou 4 atomes de carbone, -(CH₂)ᵥ-(CF₂)_{w}-CF₃, alkylcarbonyle comprenant 1, 2, 3 ou 4 atomes de carbone, alkylsulfonyle comprenant 1, 2, 3 ou 4 atomes de carbone ;
R13 signifie OH, alkyle comprenant 1, 2, 3, 4, 5 ou 6 atomes de carbone ou alcoxy comprenant 1, 2, 3, 4, 5 ou 6 atomes de carbone ;
s vaut zéro ;
t et u valent, indépendamment l'un de l'autre, zéro ou 1 ;
v et w valent, indépendamment l'un de l'autre, zéro ou 1 ;
où au moins un des radicaux R2 et R4 ne représente pas hydrogène, Cl, CN, COR13 ou -(SOᵣ)ₛ-(CH₂)ₜ-(CH₂)ₜ-CF₃ ;
R3 signifie hydrogène, F, Cl, Br, I, -CN, -NO₂, -COR14, -SO₂CH₃, alkyle comprenant 1, 2, 3, 4, 5 ou 6 atomes de carbone, alcoxy comprenant 1, 2, 3 ou 4 atomes de carbone, -Oₓ-(CH₂)_{y}-CF₃,
R14 signifie OH, alkyle comprenant 1, 2, 3, 4, 5 ou 6 atomes de carbone, alcoxy comprenant 1, 2, 3, 4, 5 ou 6 atomes de carbone ou -Oₐₐ- (CH₂)_{bb}-CF₃ ;
x vaut zéro ou 1 ;
y vaut zéro, 1, 2 ou 3 ;
aa vaut zéro ou 1 ;
bb vaut zéro, 1, 2 ou 3 ;
R5 signifie hydrogène ou F ;
ainsi que leurs sels ;
où des composés de formule I, dans laquelle R2 et R4 signifient C1 et R3 signifie F ou Cl, sont exclus,
où des composés de formule I, dans laquelle un des substituants R2 et R4 signifie Cl et l'autre des substituants R4 et R2 signifie CN et R3 signifie Cl sont exclus et
où des composés de formule I, dans laquelle R1 signifie NO₂ et les autres substituants signifient hydrogène, sont exclus.

2. Procédé pour la préparation des composés de formule I ou leurs sels, **caractérisé en ce qu'**on transforme des composés de formule II par substitution aromatique électrophile en composés de formule I, 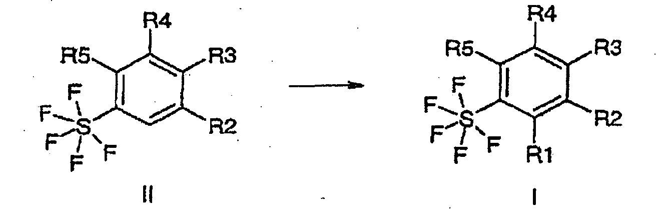 où R1 à R5 présentent la signification indiquée dans la revendication 1.

3. Composé de formule I et/ou ses sels selon la revendication 1 destiné à une utilisation comme intermédiaire de synthèse.

4. Composé de formule I et/ou ses sels selon la revendication 1 destinés à une utilisation comme intermédiaire de synthèse pour la préparation de médicaments, d'agents diagnostiques, de cristaux liquides, de polymères, de pesticides, d'herbicides, de fongicides, de nématicides, de parasiticides, d'insecticides, d'acaricides et d'arthropodicides.
